(19) Europäisches Patentamt

European Patent Office

Office européen des brevets

(11) **EP 1 384 460 B1**

(12) **EUROPEAN PATENT SPECIFICATION**

(45) Date of publication and mention
of the grant of the patent:
**31.05.2006 Bulletin 2006/22**

(51) Int Cl.:
***A61F 13/15*** *(2006.01)*

(21) Application number: **03016541.9**

(22) Date of filing: **23.07.2003**

(54) **Disposable diaper that is easy to put on a wearer in a standing position**

Im Stehen leicht anzuziehende Wegwerfwindel

Couche jetable facilement mettable sur une personne se tenant debout

(84) Designated Contracting States:
**DE FR GB SE**

(30) Priority: **26.07.2002 JP 2002218960**
**26.07.2002 JP 2002218963**
**26.07.2002 JP 2002218965**
**04.02.2003 JP 2003027632**
**04.02.2003 JP 2003027633**

(43) Date of publication of application:
**28.01.2004 Bulletin 2004/05**

(73) Proprietor: **KAO CORPORATION**
**Chuo-ku,**
**Tokyo (JP)**

(72) Inventors:
• **Okuda, Yasuyuki,**
**Kao Corporation**
**Haga-gun,**
**Tochigi (JP)**
• **Toyoshima, Haruko,**
**Kao Corporation**
**Haga-gun,**
**Tochigi (JP)**

(74) Representative: **Vossius & Partner**
**Siebertstrasse 4**
**81675 München (DE)**

(56) References cited:
| | |
|---|---|
| **EP-A- 0 391 476** | **EP-A- 0 487 921** |
| **EP-A- 0 630 631** | **EP-A- 1 184 012** |
| **EP-A- 1 226 802** | **WO-A-00/03670** |
| **WO-A-00/53140** | **WO-A-01/34083** |
| **WO-A-01/34084** | **US-A- 4 253 461** |
| **US-A- 5 451 442** | **US-A- 5 554 142** |
| **US-A- 6 123 694** | |

**Description**

The present invention relates to a fitted disposable diaper that is easy to put on a wearer, whether in a lying or standing position. It also relates to a fitted disposable diaper that is excellent in absorbency and is leak-proof.

Known disposable diapers include fitted type diapers having fastening tapes and pull-on diapers (also called pants type diapers or all-in-ones) that can be put on like ordinary underwear. Fitted disposable diapers are most commonly used because the diapers are applicable to children from newborn period, and also because of lower production cost.

Conventional fitted disposable diapers are generally designed to be put on a wearer such as a baby lying on its back. They are easy to put on a wearer who is lying still -on its back. However, an active infant hardly keeps still in a lying position during diaper changing and often resists being made to remain still in a lying position. It is not easy to keep such an active infant in a lying position during diaper changing. When in particular an infant resists being diapered in a lying position and crawls about on all fours trying to escape being diapered, it is very difficult to conduct diaper changing. It is very likely that one may try to place a diaper on an infant while in a crawling or standing position, but such attempt may be met with great difficulty.

A fitted disposable diaper that can easily be placed on a wearer in a standing position while retaining excellent absorbing performance and leakproofness is not available yet.

Under these circumstances, one may think of using disposable pull-on diapers instea.d as their baby beings pulling up and/or stands but is very likely to be frustrated because it may still be practically impossible for one to make a baby, who has just started pulling up into a standing position to raise leg in order to put on a pull-on diaper. There may be no convenient way for one to do this but to resort to changingdiapers while restraining an active baby or infant who is trying to escape from being diapered into usual lying position.

JP-A-6-63076 and JP-A-1-97201 disclose disposable diapers or underwear having an elastic band fixed to one of the longitudinal ends of the main body. In putting the diaper or underwear on a wearer, the elastic band is held around the wearer's waist, and an engageable part of the other longitudinal end is fastened to the elastic band. Since the elastic band of the proposed diaper or underwear is a closed loop, it is difficult to fit the band around the body of a standing baby by making the baby raise each leg. In short, a conventional disposable diaper cannot be seen as easy to put on a wearer whether the wearer is lying or standing.

The present invention provides a disposable diaper which includes a liquid permeable topsheet, a liquid impermeable backsheet, a liquid retentive absorbent member interposed between the topsheet and the backsheet, and the diaper having a pair of longitudinal side portions and a pair of longitudinal end sections,

a fastening tape provided on each side edge of one of the longitudinal end sections thereof and a laterally extensible side part provided on each side area of a below-waist portion of the longitudinal end section having the fastening tapes, and standing gathers provided in each of the longitudinal side portions thereof, wherein

a region R having the absorbent member in the crotch section having a low stiffness region R1 having a flexural stiffness of 25 gf/50 mm or less in the diaper width direction of the diaper,

the standing gathers being formed by fixing an elastic member having a fixing extension ratio of from 100% to 300% and the standing gathers on each longitudinal side portion having tensile characteristics, which as measured in a state of the gathers not being fixed to the diaper, wherein the tensile load required to extend the standing gathers to an effective extension ratio, which is 30% lower than a fixing extension ratio is from 20 to 120 gf, and the rate of increase of the tensile load required to extend from an extension ratio of 20% to the effective extension ratio is 1.0 gf/% or lower.

While the specification concludes with claims particularly pointing out and distinctly claiming the subject matter which is regarded as forming the present invention, it is believed that the invention will be better understood from the following description which is taken in conjunction with the accompanying drawings in which like designations are used to designate substantially identical elements, and in which:

Fig. 1 is a perspective of a disposable diaper according to an embodiment of the first aspect of the present invention;
Fig. 2 is a plan of the disposable diaper of Fig. 1 with a part cut away, in which every elastic member is stretched substantially flat;
Fig. 3 is a plan showing the stiffness design of the diaper of Fig. I;
Fig. 4 is a side view of the disposable diaper of Fig. 1 put on a standing infant with the first section B placed in the front;
Figs. 5(a) through 5(d) show other stiffness designs applicable to a disposable diaper according to a first aspect of the present invention;
Fig. 6 is a graph showing the relationship between the extension ratio and the tensile stress (load) of the standing gathers of a disposable diaper embodiment in its non-fixed state.

The present invention related to a fitted disposable diaper that is easy to put on a wearer, whether the wearer is in a lying or standing position.

The present invention also relates to a fitted disposable diaper that is easy to put on a wearer, whether in a lying or standing position, and that is excellent in absorbency and is leak-proof.

Although not wanting to be limited by theory, the present inventors have several theories as to why diapering a wearer in a standing position is difficult. One reason is that the shape of the wearer's body on the stomach side changes between a lying position and a standing position. That is, the waist is larger in a standing position than in a lying position. For example, such waist size difference of an infant can be 5cm. To facilitate diapering on an infant in a standing position, it is conceivable to increase the distance between a pair of fastening tapes on the diaper, namely the diaper width, thereby to cope with the change in waist size. In such case, however, the fastening tapes may overlap with each other when originally fixed on a wearer in a lying position.

Another reason is that one must hold the diaper onto the wearer's body while in the standing position so as to not drop the diaper while fastening the tapes. In putting a diaper on a wearer in a lying position, the diaper is placed under the weight of the wearer's back so that the diaper hardly moves out of position under such weight.

Still another reason lies in that a conventional disposable diaper is designed to have a relatively large width in its crotch portion. Whereas the crotch width of a diaper is not a large consideration regarding the ease of diapering a wearer in a lying position with its legs spread apart, a diaper with a wide crotch portion is difficult to put on a wearer in a standing position due to the difficulty in placing a diaper through a narrow space between the wearer's legs. A disposable diaper with a reduced width in the crotch portion could be placed on a wearer in a standing position with more ease, but it has been difficult to design a reduced crotch portion width without sacrificing the absorbency and leak-proof properties that are essential requirements of diapers.

Where, in particular, a fitted, disposable diaper having a pair of standing gathers (also called cuffs) and a pair of leg gathers on each longer side portions thereof is designed to have a reduced width in its crotch portion, either the distance between the pair of standing gathers or the distance between each standing gathers and the leg gathers on each side may be reduced out of necessity. Reduction of the former distance often results in insufficient absorbing performance. Reduction of the latter distance often results in the insufficient formation of a pocket between the standing gathers and the leg gathers, which can cause discharged body waste to flood over and leak.

In putting a conventional fitted disposable diaper on a wearer in a lying position, the portion having a pair of fastening tapes goes on the wearer's back-side and thus the diaper is held between the body and the bed or changing area. Therefore the other portion of the diaper where a landing zone is provided can easily be held to the wearer's stomach, and it is easy to adhere the fastening tapes, which naturally come near the landing zone, onto the landing zone. In comrast, when the diaper is put on a wearer in a standing position, one must hold the portion having the fastening tapes to the wearer's back while simultaneously fastening the fastening tapes on the landing zone of the diaper on the wearer's front side. It is very difficult for one to do this alone without help.

The present invention will be described in detail based on its preferred embodiments.

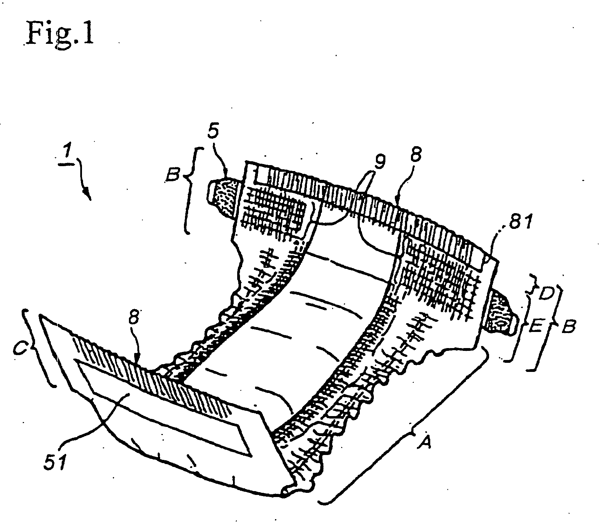

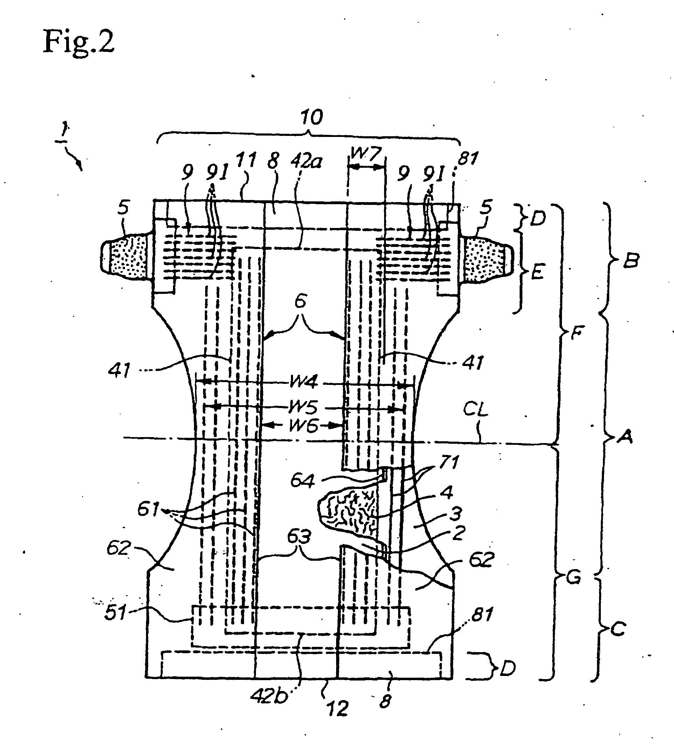

A preferred embodiment of the disposable diaper according to the present invention (hereinafter referred to as a first embodiment) is shown in Figs. 1 to 3. The diaper 1 shown in Figs. 1 and 2 comprises a liquid permeable topsheet 2, a liquid impermeable backsheet 3, and a liquid retentive absorbent member 4 interposed between the topsheet 2 and the backsheet 3. The disposable diaper 1 is of a fitted type. It is divided crosswise into a crotch section A which is in the middle lengthwise, a first section B on one side of crotch section A (one of the longitudinal end sections), and a second section C on the other side of crotch section A (the other longitudinal end section). The first section B has a pair of fastening tapes 5 provided on each side edge thereof, and the second section. C has on its outer surface a landing zone 51 for receiving the fastening tapes 5. The crotch section A is placed along the crotch of a wearer. Either the first section B or second section C may be placed on the wearer's back-side , and the other along the wearer's front (the wearer's stomach side).

The crotch section A has an inward arc on both sides thereof. The disposable diaper 1 as a whole has a sand or hour glass-shape with its longitudinal middle portion narrowed.

The topsheet 2 is substantially rectangular and larger than the absorbent member 4 when viewed from above. The absorbent member 4 is arranged in the middle of the width of backsheet 3. The backsheet 3 is sand or hour glass-shaped in agreement with the contour of diaper 1.

The topsheet 2 and the backsheet 3 both extend outward from the two longer sides 41 and two shorter sides 42a and 42b of the absorbent member 4 and are bonded together at these extensions. The backsheet 3 extends outward from the two longer sides of the topsheet 2.

A pair of standing gathers (also called cuffs) 6 is lengthwise provided on both longitudinal side portions of the diaper 1. The standing gathers 6 on each side are formed of a gather-forming sheet 62 having elastic members 61, the gather-forming sheet 62 being disposed to cover both side areas of the longer side edge of the topsheet 2. Each sheet 62 is fixed to the topsheet 2 in a straight line pattern parallel to the diaper longitudinal direction by known bonding means such as a heat seal or an adhesive. The straight line is positioned between the longer side edge 41 of the absorbent member 4 and the laterally most inward leg elastic member 71(described later). The linear joint of the sheet 62 with the topsheet 2 is a fixed end 64 of the standing gathers 6. The part of the sheet 62 extending outward from the fixed end 64 is fixed to the topsheet 2 or the backsheet 3. In the areas near the two longitudinal ends of the diaper 1, the part of the sheet 62 extending inward from the fixed end 64 is fixed to the topsheet 2. The elastic members 61 used to form the standing gathers 6 are elastic threads, which are arranged per side substantially in parallel with the free end 63 of the standing gathers 6.

The first section B has a waist portion D and a below-waist portion E. An elastic member 81 is provided in the waist portion D to form an extensible waist part 8. A plurality of elastic members 91 are provided on each side area of the below-waist portion E to form a pair of laterally extensible side parts 9. Both of the extensible side parts 9 are arranged in the area between the pair of fastening tapes 5.

The second section C also has an extensible waist part 8 in its waist portion D. The two waist portions D constitute the longitudinal end parts of the diaper 1 and are applied to the wearer's waist. More specifically, an area from the longitudinal end 11 or 12 to 20 mm inward is called a waist portion D. The diaper 1 according to a first embodiment has the extensible waist part 8 formed by the waist elastic member 81 in the waist portion D in both the first and second sections B and C. The waist elastic member 81 in each waist portion D is fixed between the backsheet 3 and the topsheet 2 or the sheet 62 in its stretched state across the diaper. The waist elastic member 81 used in this embodiment is a strip of urethane foam.

Fig. 2 is a plan view of the disposable diaper 1 with all its elastic members in their stretched state; The upper part of the diaper 1 is designated first section B, and the lower part second section C. The below-waist portion E of the first section B is a portion located below the waist portion D (20 mm wide from the top end 11 downward) and above the crotch section A (the portion applied to the crotch of a wearer and describing an inward arc on both sides thereof in conformity with the wearer's thighs). The plurality of below-waist elastic members 91 are arranged per extensible side part 9 in parallel to the width of the diaper at prescribed intervals.

The below-waist elastic members 91 are fixed in their stretched state such that elastic extensibility may develop in at least areas extending outward from both side edges 41 of the absorbent member 4 in the direction of the width of the diaper. The below-waist elastic members 91 are not disposed in the middle portion of the area between the longer side edges 41 of the absorbent member 4. The elastic members 91 are fixedly sandwiched in between two soft sheets (not shown) which are provided in the first section B over the width of the main body 10 (see Fig. 2). The term "main body 10 of the diaper 1" as used herein means the part composed of the topsheet 2, the backsheet 3, and the absorbent member 4 and including all sections A to C. The two sheets are fixed by bonding between the backsheet 3 and the topsheet 2 or the gather-forming sheet 62. The inward end of each below-waist elastic member 91 is positioned slightlly inward from the side edge 41 of the absorbent member 4. Therefore, there is no elastic; member 91 placed in the area from the middle of the width of the absorbent member 4 up to a position near each side edge 41.

The diaper 1 also has a pair of leg gathers on each longitudinal side thereof The leg gathers on each side are formed by arranging a plurality of leg elastic members 71 in parallel with the diaper length.

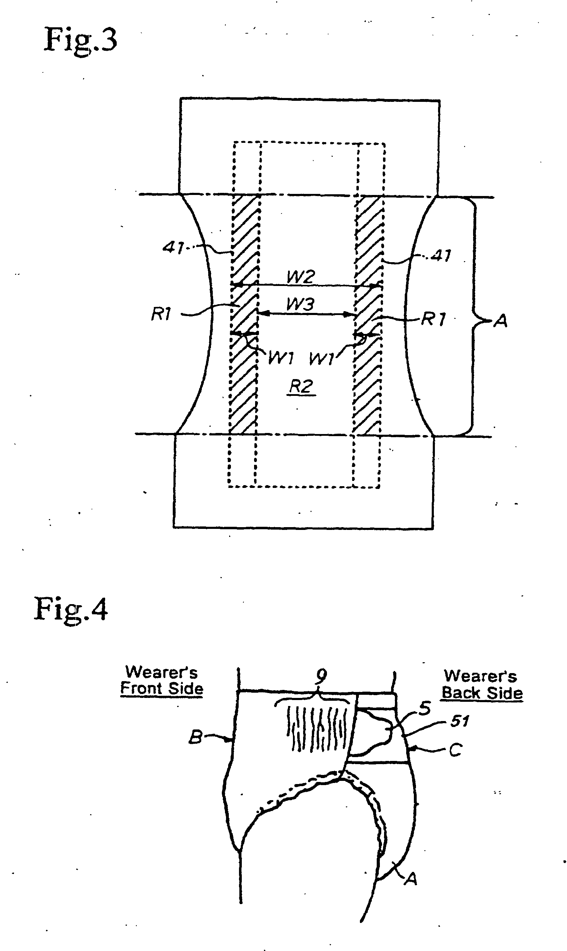

As shown in Fig. 3, the diaper 1 according to a first embodiment is characterized by regions R1 having a lateral flexural stiffness of 25 gf/50 mm or lower in the region having the absorbent member 4 in the crotch section A. More specifically, in the crotch section A, the region where the absorbent member 4 is disposed (hereinafter referred to as the region R) contains regions R1 indicated by slant lines in Fig. 3 which are less stiff (softer) than the other region R2 in the region R The region R1 will hereinafter be called a low stiffness region R1. The low stiffness regions R1 have a lateral (in the diaper width direction) flexural stiffness of 25 gf/50 mm or lower. The term "region" as in "the region having the absorbent member 4 in the crotch section A (region R)" or as in "the low stiffness region" as used herein means a three-dimensional part containing the absorbent member 4 in its thickness (between the upper and lower surfaces of the diaper).

In a first embodiment, each of the low stiffness regions R1 is an oblong region provided along each longer side edge of the absorbent member 4.

The region R in the crotch section A is required to have sufficient width and space for collecting and retaining body waste within the crotch section while being worn and should have a minimum restoring force for maintaining such width and space. In diapering a baby in a standing position, when two ends of the diaper that is passed between legs are pulled up in the front and the back, it is desirable for the diaper itself to fit the crotch snugly and not to slide down, in order to facilitate fastening. Considering these factors, the lower limit of the flexural stiffness of the low stiffness region R1 is preferably about 3 gf/50 mm, still more preferably about 5 gf/50 mm. For assuring ease of diapering without sacrificing fit and safety against leakage, a preferred flexural stiffness of the low stiffness region R1 is 25gf150mm or lower, more preferably from 3 to 25 gf/50 mm, and even more preferably from 5 to 20 gf/50 mm.

In the diaper 1 of a first embodiment, the region R also has a region R2 having a width W3 (see Fig. 3) of preferably at least 50 mm across the diaper and a lateral flexural stiffness of higher than 25 gf/50 mm (hereinafter referred to as a high stiffness region R2). From the standpoint of ease of diapering and shape retention during use, a preferred upper limit of the flexural stiffness of the high stiffness region R2 is 60 gf/50 mm, and a more preferred flexural stiffness of the high stiffness region R2 is from 30 to 50 gf/50 mm.

To improve ease of diapering a wearer in a standing position, the width W1 (see Fig. 3) of each low stiffness region R1 preferably ranges from 15 to 50% of the width W2 (see Fig .3) of the region R, and the total width of the low stiffness regions R1 preferably ranges from 30 to 100% of the width W2. The width W3 (see Fig. 3) of the high stiffness region R2 between the pair of the low stiffness regions R1 is preferably from 0 to 70% of the width W2. The width W2 is preferably 70 mm or longer in order to secure sufficient absorbency of the crotch section. The region R width W2 of 70 mm or longer is also effective in facilitating the fastening of a diaper while a wearer is in a standing position because, when the diaper is inserted between 'the wearer's legs and pulled upwards, the crotch section fits the wearer's crotch well and does not readily slide down thereby making it easy for one to fasten tapes. From the standpoint of ease of diapering and proper fit to the wearer's crotch, the width W2 is preferably 150 mm or shorter. The width W3 is preferably from 0 to 80 mm. The smaller the width W3, the better for ease of diapering and obtaining a snug fit.

The lateral (in the width direction of the diaper) flexural stiffness of the low stiffness region R1 and the high stiffness region R2 is measured as follows.

Measurement of flexural stiffness:

Standing gathers are removed from a diaper. A 50 mm wide and 80 mm long rectangular specimen containing all the constituent members including from the topsheet to the backsheet in its thickness direction is cut out of the region R. The width (50 mm) and the length (80 mm) of the specimen are in the length and the width directions of the diaper, respectively. Care should be taken so that the specimen has substantially no crease or wrinkle that may influence the measurement.

Measurement is carried out with a Tensilon tester, RTC-1150A supplied by Orientec, equipped with a 5-kg load cell (range: 200 gf) in accordance with JIS K7171 (plastics-Determination of flexural properties) (R1=5.0±0.1 mm, R2=±0.2 mm). The specimen is placed on two supports 50 mm apart with its length being along the line connecting the two supports, and an indenter is positioned to just touch the mid-point of the specimen. The indenter is moved down at a speed of 30 mm/min to obtain a load-deformation curve. The maximum of the bending stress is taken as the flexural stiffness (gf/50 mm). The "50 mm" in the unit corresponds to the width of the specimen bent by the indenter.

Where the region R1 or R2 is greater than 50 mm across the diaper, a specimen is cut such that the whole width of the region is contained in the specimen length. Where the region R1 or R2 is equal to or shorter than 50 mm across the diaper, a specimen is cut out so as to contain the whole width of the region in the specimen length and is placed over the supports with at least part of that region positioned between the supports. For instance, where a low stiffness region R1 having a width of 50 mm or smaller is arranged on each side of the region R, a specimen of the region R1 is cut out such that the low stiffness region R1 is present in one longitudinal end portion of the specimen and placed on the supports such that one end of the low stiffness region is positioned at the edge of one support and the other end between the supports. In this case, the mid-point of the specimen, at which the indenter presses, may be outside of the low stiffness region R1. Nevertheless the measured maximum load is regarded as the flexural stiffness (gf/50 mm) of the low stiffness region because the maximum load is predomninantly governed by the stiffness of the low stiffness region.

Where a specimen containing a region whose width exceeds 50 mm in the diaper width direction shows a maximum load exceeding 25 gf/50 mm as measured in the same manner as described above, the region R has a high stiffness region R2 having a width W3 (see Fig. 3) of 50 mm or larger and a lateral flexural stiffness exceeding 25 gf/50 mm.

The low stiffness region R1 in a first embodiment is formed by varying the constitution of part of the absorbent member 4 within the region R from the constitution of the other part, that is, by reducing the basis weight of part of the absorbent member 4 as compared to that of the other part.

Methods of reducing the basis weight of part of an absorbent member from that of the other part include the following ; (1) An absorbent member is fabricated of an upper absorbent layer and a lower absorbent layer, part of which is made solely of either the upper or the lower absorbent layer to provide a low stiffness region; (2) An absorbent member is fabricated of fibers air-laid by suction on a net, part of which has a smaller air-laid fiber content than the other part; (3) An absorbent member is fabricated of three or more absorbent layers, part of which has a smaller number of absorbent layers by at least one layer from the other part; (4) An absorbent member is fabricated of two or more absorbent layers, part of which lacks one or more constituents. Preferred is method (1) because the upper and lower absorbent layers are separately prepared, which is advantageous in realizing the absorbing performance and the basis weight as designed

In addition to making a difference in the basis weight, the low stiffness region can also be formed by other methods, such as (a) slitting the absorbent member 4, (b) discretely arranging separate pieces of an absorbent member, (c) subjecting part of an absorbent member to embossing or like treatment, (d) disposing or incorporating fibers having compressive elasticity, (e) eliminating an adhesive (e.g., a hot-melt adhesive), and (f) eliminating one or more members making up the diaper.

The standing gathers 6 are formed by fixing a low modulus elastic member at a high extension ratio. The extension ratio at which an elastic member is fixed will hereinafter be referred to as a fixing extension ratio. In the present embodiment, the elastic member for standing gathers is fixed in a stretched state at a fixing extension ratio of 100% or higher, preferably from 100 to 300%, more preferably from 130 to 200%.

If the fixing extension ratio of the elastic member is less than 100%, the resultant gathers do not rise up sufficiently in a fitted diaper. It follows that the standing gathers 6 may fall or readily bend when the free end of the standing gathers 6 is held to the wearer's crotch particularly in diapering a wearer in a standing position, making correct diapering difficult. Besides, when a wearer changes its position, a gap may be formed between the free end of the gathers and the skin, through which body waste can leak.

If the fixing extension ratio is more than 300%, the disposable diaper may curl up excessively and be difficult to put on. Such a curling diaper may be particularly difficult to put on a wearer in a standing position because the diaper put through between the legs wilt slide down easily before it can be fastened. If the diaper has already begun to slide down when one manages to fasten the diaper, this results in poor fit and impaired leak-proof properties. As long as the fixing extension ratio is 300% or less, the diaper is preferably prevented from curling up and is easy to put on. In diapering a wearer in a standing position, since the diaper hardly slides down between legs while fastening with the fastening tapes, the diaper can easily be put on in the right position without bunching to provide a good fit and have satisfactory leak-proof properties.

The fixing extension ratio of the standing gathers 6 is measured as follows.

Measurement of fixing extension ratio:

A strip of the gathered forming sheet 62 having an elastic member is cut off along the diaper length direction so that the strip (specimen) may contain at least the lengthwise middle of the gathers. The specimen is stretched to the maximum, i.e., to the stretched state as shown in Fig. 2. A distance between arbitrarily chosen two points, preferably about 200 mm apart, of the stretched specimen is taken as a stretched length H. The specimen is then put into its natural state, i.e., let to contract spontaneously, and a distance between the same two points (natural length h) is measured. The fixing extension ratio is represented by equation (1):

$$\text{Fixing Extension ratio (\%)} = [(H-h)/h] \times 100 \qquad (1)$$

The natural length h is measured by placing the gathered strip on a flat surface while minimizing the unevenness due to gathering with minimized load applied thereto.

The tensile characteristics of the standing gathers 6 (either one of the pair) are such that the tensile load required to extend the standing gathers 6 in its non-fixed state (the state not fixed to the diaper, that is, the state after being cut off the diaper) to an effective extension ratio (equivalent to the value which is 30% lower than the fixing extension ration) is from 20 to 120 gf, preferably from 50 to 100 gf, and that the increase rate of tensile load required for extending in the non-fixed state from an extension ratio of 20% up to the above-identified effective extension ratio is 1.0 gf/% or lower, preferably 0.7 gf/% or lower.

Although not wanting to be limited by theory, the reason why the tensile characteristics of the standing gathers are evaluated at the effective extension ratio, (e.g. the fixing extension ratio - 30%), is that the extension (%) vs. load curve often contains the tensile load component attributed to the constituent members of the standing gathers in the region above the effective extension ratio. The part of the curve above the effective extension ratio is likely to represent a tensile stress in excess of the physical properties of the gathers *per se,* that is, false data. In addition, a fitted diaper with standing gathers is curved into a U-shape when put on, and the free end of the standing gathers disposed on the inner side of the diaper depicts a U-shape having a shorter length than that of the outer surface of the U-shaped diaper. Since the standing gathers are rarely stretched to the fixing extension ratio in actual use, it can be seen as reasonable to discuss the behavior of the standing gathers at a given extension ratio lower than the fixing extension ratio in evaluating the behavior during an actual use.

The tensile characteristics (tensile load and tensile load increase rate) of the standing gathers at the effective extension ratio in a non-fixed state are measured as follows.

Measurement of tensile characteristics:

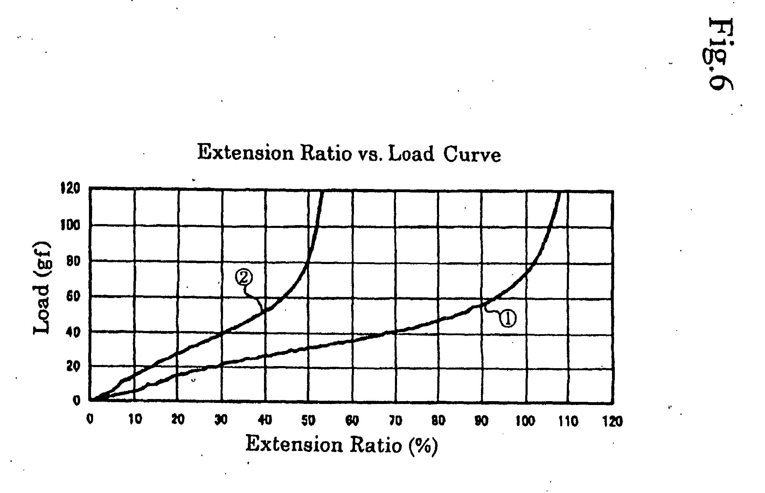

A strip of the gathered sheet 62 having an elastic member is cut off at its fixed end 64 from the part containing at least the lengthwise middle of the standing gathers 6 to prepare a specimen. The specimen should have a natural length of at least 70 mm as measured on a flat surface while minimizing the unevenness due to gathering with minimized load applied thereto. It is desirable to use the same specimen used in the measurement of the fixing extension ratio. The specimen is clamped in the jaws of a Tensilon tensile tester, RTC-1150A supplied by Orientec, equipped with a 5 kg-load cell. The jaws are set at an initial distance of 50 mm, which is an initial natural length of the specimen at 0% extension. The specimen is extended up to the fixing extension ratio at a rate of 300 mm/min, and the extension ratios vs. increasing tensile load are plotted to obtain a curve with the extension ratio (%) as abscissa and the tensile load (gf) ordinate (see Fig. 6).

The tensile load at an extension ratio of 20% and an effective extension ratio are read from the curve. The slope of the curve of from the extension ratio of 20% to the effective extension ratio, represented by (tensile load at effective extension ratio - tensile load at 20% extension ratio)/(effective extension ratio (%) - 20%), is calculated to obtain a tensile load increase rate (gf/%) of from 20% extension ratio to effective extension ratio.

If the tensile load at the effective extension ratio is less than 20 gf, the gathers do not rise up sufficiently in a fitted diaper. It follows that the standing gathers may fall or readily bend when the free end of the standing gathers is held to the wearer's crotch particularly in diapering a wearer in a standing position, making correct diapering difficult. Besides, when a wearer changes position, a gap may be formed between the free end of the gathers and the skin, through which body waste can leak. Even when the free end of the pair of standing gathers is in close contact with the wearer's body, the squeezing force of the standing gathers is so weak that the force of shutting discharged body waste in the space between the pair of standing gathers would be insufficient. It is likely to follow that the body waste edges out over the free end of the standing gathers. Therefore, the standing gathers with such a low tensile load cannot be seen as effective against leakage.

If the tensile load at the effective extension ratio is more than 120 gf, the U-shaped diaper needs a large force to open the diaper wide and may, be difficult when placing a diaper on a wearer lying on its back When changing diapers after being soiled, the diaper is difficult to handle due to its strong tendency to curl up. Furthermore, in diapering a wearer in a standing position, the diaper placed between the legs may roll up or slide down, -making fastening difficult.

These problems occur when the elastic member is fixed at a high extension ratio as with the case of embodiments of the present invention. In other words, the inconvenience arises from the phenomenon that the standing gathers stretched in diapering exhibit contractibility to be relieved from the tensile stress and make the whole diaper roll-up. Solving such problems, the present invention has succeeded in assuring ease of diapering by reducing the tensile load to some extent thereby suppressing its behavior to contract, retarding the contraction, or reducing the force necessary to widely open the contracted gathers. With the fixing extension ratio being equal, the tensile stress is a governing factor., The smaller the tensile stress, the more extensible the standing gathers and the easier to handle the diaper. Unless the tensile load at the effective extension ratio exceeds 120 gf, the manageability of the diaper is not impaired, and one is able to widely open the diaper with ease.

The tensile load increase rate is desirably as small as possible. With a smaller increase rate, the force of the standing gathers to come into intimate contact with the skin and to block body waste will be less variable depending on the wearer's position or the way of diapering. Further, the diaper will be easier to handle, giving no feeling of abrupt change in force necessary to widely open the diaper (same as widely open). As a result, the diaper will be easier to put on whether a wearer is in a lying position or a standing position.

Accordingly, it is desirable that the tensile load increase rate, calculated as an increase in tensile load per unit increase in extension ratio, be 1.0 gf/% or lower at every point between 0% extension ratio up to an effective extension ratio. The tensile load increase rate may exceed 1.0 gf/% in some regions between 0% extension ratio up to an effective extension ratio. A diaper showing such a tensile load increase rate as exceeding 1.0 gf/% is not deemed to be a departure from the scope of the present invention as long as such a high increase rate is observed in the region of from 0% extension ratio to 20% extension ratio. That said, the upper limit of the tensile load increase rate in the region from 0 to 20% extension is preferably 2.0 gf/%.

This is the reason the tensile load increase rate in the region from 20% extension to an effective extension ratio is specified in the present invention.

The disposable diaper 1 according to a first embodiment has a low stiffness region R1 as part of the region R (the region having the absorbent member 4 in the crotch section A) and a pair of low-modulus standing gathers 6 provided at a high extension ratio. This design renders the diaper 1 extremely easy to put on a wearer in a standing position as well as a lying position.

A conventional fitted type disposable diaper is relatively easy to put on a wearer (e.g., a baby) lying on its back because one can easily spread the wearer's leg wide. In diapering a wearer in a standing position, on the other hand, since it is difficult to spread the wearer's leg wide while standing up , one must place a disposable diaper, which is made up of so many members, through a narrow space between the legs. Probably because of this, the position of a disposable diaper that has been put on a standing wearer is mostly below the intended position. In other words, the front waist size of the diaper that has been put on a wearer when the wearer is standing is larger than the true front waist size of the wearer. One has difficulty in diapering a wearer in a standing position with a conventional fitted type disposable diaper as compared with diapering a wearer in a lying position because of not only the change in the front waist size of a wearer depending on whether the wearer is standing or lying but also the change of the front waist size of the diaper with the change of the position where the waist portion of the diaper is held against.

To solve this problem, it might be considered effective to reduce the lateral stiffness of a disposable diaper. However, a diaper so designed undesirably bunches up in its crotch portion and may leak. Therefore, such an approach seems to be impractical.

According to a first embodiment, the standing gathers preferably have a high extension ratio and a low modulus and therefore rise sharply and firmly. Even if the crotch portion bunches, the standing gathers secure space enough to substantially prevent leakage. The diaper of this embodiment can be smoothly placed through the narrow space between the legs of a wearer in a standing position and held to the body at the same correct position as in diapering a wearer in a lying ,position. Thus, the present embodiment is both leak-proof and easier to diaper a wearer in a standing position. With the ease of diapering a standing wearer being so improved, it is no longer necessary to largely increase the extension length of the first section B or the width of the first section B, so that there is no impairment of ease in diapering a wearer in a lying position.

The standing gathers of the present invention can be formed by, for example, adhering at least one elastic member 61 to a gather-forming sheet 62, such as a nonwoven fabric. The elastic member or members have a tensile load increase rate of 1.0 gf/% or less from 20% extension ratio to an effective extension ratio and a tensile load of from 20 to 120 gf at the effective extension ratio as a whole. The term "as a whole" as used herein means "as a total of two or more elastic members". The elastic members for standing gathers include elastic threads (e.g., rubber threads, preferably those having a thickness of 450 dtex or smaller), elastic strips (e.g., rubber strips, preferably those having a thickness to width ratio of 0.1 to 1), and elastic films. Materials of the elastic members include natural rubber, synthetic rubbers (e.g., styrene-butadiene, butadiene, isoprene or neoprene rubbers), ethylene-vinyl acetate copolymers, extensible polyolefins, and urethane rubber. In using a plurality of elastic members, it is preferred for each of them to have a tensile load increase rate of 0.5 gf/% or lower from 20% extension ratio to the respective effective extension ratio and a tensile load of 5 to 50 gf at the effective extension ratio.

The disposable diaper 1 of the first embodiment has the below-waist elastic ntemlbers 91 fixedly held between two sheets integrated with the main body 10 of the diaper 1. The main body 10 is thus provided with elastic extensibility as desired. Unlike the configurations in which a separately prepared elastically extensible member is joined to each side of the main body of a disposable diaper as disclosed in JP-T-9-507409 and JP-A-6-63077, the extensible side parts are prevented from being broken even when the fastening tapes 5 are pulled strongly in cases, for example, where one hastily places a diaper on an infant crawling about and trying to escape being diapered. The diaper 1 is therefore easy to put on a wearer either from the front or the back of the wearer. That is, one can put the diaper 1 under the back of a wearer lying on its back (facing the person placing the diaper) and adhering the fastening tapes 5 to the landing zone 51 on the stomach side (this way of diapering will be sometimes called "a back-to-front way of diapering"), or one can put on the diaper 1 backward and adhering the fastening tapes on the back side of the wearer (this way of diapering will be sometimes called "a front-to-back way of diapering").

In the disposable diaper 1 of the first embodiment, two portions on opposite sides of the centerline CL (see Fig. 2) dividing the length of the diaper 1 in equal halves, designated as sections F and G, preferably have a saturated absorption capacity ratio of from 45/55 to 55/45. By so designing, the diaper 1 does not substantially leak even when put on in a front-to-back way of diapering. The diaper 1 is not only suited to a back-to-front way of diapering but also capable of being put on in a front-to-back way with ease and does not leak whichever way of diapering is chosen. In order to substantially prevent leakage irrespective of the way of diapering, the F/G ratio of saturated absorption capacity preferably ranges from 48/52 to 52/48, and the difference in saturated absorption capacity between the F and G sections is preferably as small as possible. In Fig. 4 is shown the disposable diaper 1 put on an infant in a standing position with the first section B on the stomach (in a front-to-back way).

The saturated absorption capacity of the section on each side of the centerline CL is measured as follows.

Method of measuring saturated absorption capacity:

A diaper 1 is cut across into two along the centerline CL, the line dividing the length of the diaper into equal halves: The standing gathers, the leg gathers, and the extensible side parts in the below-waist portion are cut off from each half. Care should be taken to keep the structure of the absorbent member intact. The cut piece is placed on a metal net which is configured to be taken out of a container horizontally. The metal net with the cut piece on is weighed and then put in a container having a drain hole in the lower part of one side thereof. The drain hole is closed, and 0.9 wt% physiotogical saline is poured to completely immerse the absorbent member at a rate controlled, so that polymer particles and the like may not fall off from the cut edge of the diaper. After completely immersed, the cut piece is allowed to stand for 30 minutes. The container is tilted 10° with the drain hole side down, and the drain hole is opened to discharge the liquid. The container is kept tilted for 30 minutes until the liquid is completely drained. The metal net with the cut piece on is taken out of the container and weighed again. The difference between the weight after immersion and the weight before immersion is taken as the saturated absorption capacity (g) of the cut piece.

Each of the extensible side parts 9 preferably has a natural length of 20 mm or more, more preferably from 20 to 100 mm, as measured in the diaper width direction in the state removed from the diaper and contracted. The tensile characteristics of the pair of extensible side parts 9 are preferably such that, when the fastening tapes 5 are pulled apart in the diaper width direction to extend the extensible side parts 9, the tensile load at a middle extension length is 30 to 300 gf. The tensile load at a middle extension length is measured as follows. The fastening tapes are clamped in the jaws with the first section B being in its natural state (i.e., contracted state) and pulled to extend the first section B to a length of 95% of the maximum extension, length. The tensile load required to extend to the mid-point between the natural length and the maximum extension length is read.

The diaper 1 is easy to put on since the natural length of each extensible side part 9 in the diaper width direction is 20 mm or greater. In particular, it is easy to fix the fastening tapes to the landing zone in putting the diaper 1 on a wearer in a standing position and a wearer not facing the person trying to place the diaper. The diaper 1 is capable of coping with the change in front waist contour and size to impose a moderate squeezing force.

Where the tensile stress at the middle extension length of the first section B is 30 gf or greater, the diaper 1 offers a good fit to the wearer's below-waist portion. The diaper is prevented from sliding down, losing its shape, or making a gap through which body waste may leak and clothes may enter. Where the tensile stress at the middle extension length is 300 gf or smaller, the squeezing force is controlled moderately such that a wearer does not feel tightness. The extensible side parts can be extended by a moderate force, which makes diapering easy. This is particularly advantageous for diapering a wearer in a standing position or adhering the fastening tapes to the landing zone on the back of a wearer who is crawling.

To ensure the improvement of ease to put the diaper on a wearer in a standing or lying position, especially a wearer in a standing position, it is preferable that the smallest width W4 (see Fig. 2) of the diaper 1 in the crotch section be from 100 to 240 mm, preferably from 120 to 230 mm, still even more preferably from 120 to 220 mm, and that the distance W5 (see Fig. 2) between the leg elastic member 71 that is the most outward on one side of the diaper and that on the other side preferably be from 90 to 230 m, more preferably from 100 to 220 mm, still even more preferably from 100 to 200 mm. To assure ease of putting the diaper between the wearer's legs and pulling the diaper upward, it is preferred that the distance W6 (see Fig. 2) between the free ends of the paired standing gathers 6 is 100 mm or smaller, particularly 80 mm or smaller. The width W7 (see Fig. 2) of the standing gathers on each side is preferably 25 to 60 mm.

With considerations given only to diapering a wearer in a lying position, the narrowing of the width of the crotch section, e.g. the target zone where urine is going to be provided, leads to leakage. In the first place, a wide crotch section is no hindrance to diapering a lying wearer. Diaper manufacturers would not generally think of narrowing the crotch width. On the other hand, the present inventors have revealed the need of disposable diapers that is easy to put on not only a lying baby but a standing baby without raising the baby's leg one by one. In other words, the inventors have studied disposable diapers suited to those wearers who have mastered crawling and are able to pull themselves up on furniture and walk around holding on to it and are now moving on to become toddlers. To meet this need, the inventors have reached an idea that the crotch width could be reduced for improving ease of diapering. This idea has been realized by providing low modulus standing gathers for securing high safety against leakage while being worn. The inventors have thus succeeded in improving the ease of diapering a wearer in a standing position without sacrificing leakage, whether the diaper has been put on a wearer in a standing position or lying position

Materials of the members making up the disposable diaper 1 according to a first embodiment will be described below.

The materials for forming the topsheet 2, the backsheet 3, the standing gathers-forming elastic members 61, the standing gather-forming sheet 62, and the landing tape for forming the landing zone 51, and the like are not limited and selected appropriately from known materials commonly used in conventional disposable diapers.

The absorbent member 4 includes one made of a fiber aggregate and one comprising a fiber aggregate and a superabsorbent polymer. The fiber aggregate includes nonwoven fabrics or fiber webs prepared by various processes. In using a superabsorbent polymer, the polymer may be dispersed in the interstices of a fiber aggregate or sandwiched in the form of a layer between nonwoven fabrics or fiber webs made of fibrous materials. The absorbent member comprising a fiber aggregate or a combination of a fiber aggregate and a superabsorbent polymer is preferably wrapped in a soft sheet of paper or liquid-permeable nonwoven fabric.

The fastening tape 5 includes a tape having a male member (hook) of a mechanical fastener and a tape having a self-adhesive layer formed by applying a self-adhesive. It is likely that a fastening tape with a self-adhesive layer sticks to a one's hand, making it difficult to carry out fastening in a front-to-back way. To avoid this, fastening tapes that can be mechanically engaged on the landing zone 51, such as those having a male member of a mechanical fastener, are preferred especially for a front-to-back way of diapering. Where the outer surface of the backsheet 3 is made of an engageable material such as a nonwoven fabric, that surface can serve as a landing zone 51 for the mechanical fasteners.

The waist elastic members 81, the below-waist elastic members 91, and the leg elastic members 71 are known and conventional. Useful forms of these elastic members include threads (e.g., rubber threads), tapes or strips of a certain width (e.g., rubber strips), and films. Materials of the elastic members include natural rubber, synthetic rubbers (e.g., styrene-butadiene, butadiene, isoprene or neoprene rubbers), ethylene-vinyl acetate copolymers, extensible polyolefins, and urethane rubber.

The waist elastic member 81 is preferably an elastic strip having a predetermined width. The below-waist elastic members 91 are preferably elastic threads. The leg elastic members 91 are preferably rubber strips. The number of the elastic threads 91 per extensible side part 9 is preferably about 3 to 12.

While the present invention has been described with particular reference to its preferred embodiments, it should be understood that the invention is not construed as being limited thereto, and various modifications can be made therein without departing from the scope thereof.

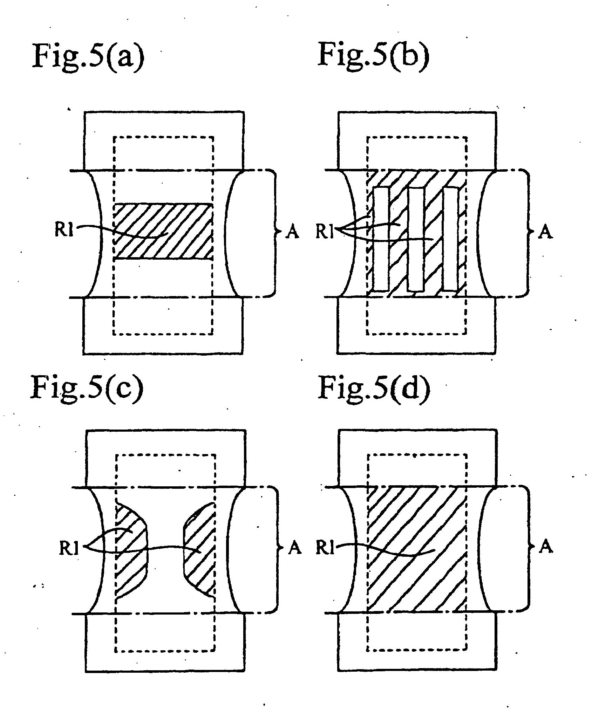

For example, the following modifications can be made to a first embodiment. While the diaper 1 has the low stiffness region R1 discretely provided on both sides of the region R in the diaper length direction, the low stiffness region R1 can be arranged according to the configurations shown in Figs. 5(a) to 5(d). Fig. 5(a) shows a configuration in which a low stiffness region R1 is continuously provided in the middle portion of the region R over the whole width of the absorbent member 4. Fig. 5(b) depicts a configuration in which a low stiffness region R1 is provided all over the region R except for discretely arranged high stiffness regions. Fig. 5(c) shows another stiffness configuration. Fig. 5(d) displays a configuration in which all the region R is a low stiffness region R1.

While the diaper 1 has an extensible part on each side area of the below-waist portion E of the first section B (a pair of side extensible parts 9), a laterally extensible part may be additionally provided between the pair of side extensible parts 9 in the below-waist portion E either as an extension from the side extensible parts 9 or discretely from the side extensible parts 9.

The leg elastic members 71 may be arranged to depict a curve along each arched side edge of the crotch section A. The sections, portions, parts, and the other members constituting the disposable diaper of the present invention are also subject to variation in shape or configuration.

The disposable diaper according to the present invention is, while applicable to a wide range of wearers from newborns to adults, is particular suited for babies and iafants, especially those who are prone to hate being diapered and those who have the ability to pull themselves up into a standing position.

In the description given above, some particulars of an embodiment may have been omitted for the sake of avoiding redundancy. This omission can be complemented by the corresponding description of other embodiments. Particular characteristics of an embodiment may be applied to other embodiments appropriately. Particulars of every embodiment are appropriately interchangeable between embodiments.

The present invention will now be illustrated in greater detail with reference to. Examples, but it should be understood that the invention is not construed as being limited thereto.

Examples of the invention:

EXAMPLE 1

A disposable diaper shown in Figs. 1 to 3 was prepared. In Table 1 are shown the smallest width W4 in the crotch section A, the width W2 of the region R, the width W3 and flexural stiffness of the high stiffness region R2, the width W1 and bending stiffness of each of the low stiffness regions R1, the fixing extension ratio of the standing gathers, the tensile characteristics (i.e., effective extension ratio (= fixing extension ratio-30%), tensile load at the effective extension ratio, and tensile load increase rate) of the standing gathers in their non-fixed state, and the section F to section G ratio of saturated absorption capacity. The standing gathers on each side were formed of two elastic strips adhered to a nonwoven fabric sheet.

The absorbent member was fabricated by the aforementioned method (1). The absorbent member had a, stiff part in the widthwise middle and a soft part on each longer side thereof. The disposable diaper prepared had otherwise the same members in the same configuration as in a standard product hereinafter described.

EXAMPLE 2

A disposable diaper was prepared in the same manner as in Example 1, except for changing the width W3 and bending stiffness of the high stiffness region R2 and the width W1 and bending stiffness of the low stiffness region R1 as shown in Table 1.

EXAMPLES 3 AND 4

Disposable diapers were prepared in the same manner as in Example 1, except for using an absorbent member having a low bending stiffness all over the entire area, the width and the bending stiffness of which are shown in Table I.

All the diapers of Examples 1 to 3 had the following properties. The lateral tensile load of the below-waist portion E in the first section B at a given extension ratio was greater than that of the waist portion D in the first section B at the same extension ratio. Each of the side extensible parts measured about 40 mm in the diaper width direction in its contracted state. When the two fastening tapes were pulled apart in the diaper width direction to extend the extensible side parts, the tensile load at a middle extension length ranged from 30 to 150 gf.

Standard product:

A commercially available fitted type disposable diaper (Merries Morenai-Fit Nobichizimi Gather, Size M for infants, available from Kao Corp.) was used as a standard product for comparison. The dimensional and physical characteristics of this product are shown in Table 1. When the fastening tapes were pulled apart in the diaper width direction, the tensile load at a middle extension length ranged from 30 to 150 gf.

COMPARATIVE EXAMPLE 1

A disposable diaper having the same structure as the standard product was prepared, except for using an absorbent member having a stiff part in the widthwise middle and a soft part in each longer side thereof.

COMPARATIVE EXAMPLE 2

A disposable diaper having the same structure as the standard product was prepared, except for using an absorbent member having a high stiffness all over the entire area thereof.

COMPARATIVE EXAMPLE 3

A disposable diaper was prepared in the same manner as in Example 3, except that no elastic member was disposed in the below-waist portion.

Fig. 6 is a graph showing the relationship between the extension ratio and tensile stress (load) of standing gathers in their non-fixed state. Curve 1 represents the diapers of Examples 1 to 3, and curve 2 represents the standard product.

TABLE 1

| | W4 (mm) | W2 (mm) | Region R2 | | Region R1 | | Fixing Extension Ratio (%) | Non-fixed State Tensile Characteristics | | | F/G Saturated Absorption Capacity Ratio |
|---|---|---|---|---|---|---|---|---|---|---|---|
| | | | W3 (mm) | Bending Stiffness (gf/cm) | W1 (mm) | Bending Stiffness (gf/cm) | | Effective Extension Ratio (%) | Tensile Load at Effective Extension Ratio (gf) | Tensile Load Increase Rate (gf%) | |
| Standard Product | 244 | 100 | 100 | 28.4 | - | | 60 | 30 | 40 | 1.2 | 45/55 |
| Example 1 | 220 | 100 | 70 | 44.5 | 15 | 21.2 | 110 | 80 | 48 | 0.5 | 50/50 |
| Example 2 | 220 | 100 | 60 | 44.5 | 20 | 21.2 | 110 | 80 | 48 | 0.5 | 50/50 |
| Example 3 | 220 | 100 | - | | 100 | 6.2 | 110 | 80 | 48 | 0.5 | 50/50 |
| Example 4 | 220 | 100 | - | | 100 | 24.0 | 110 | 80 | 48 | 0.5 | 50/50 |
| Comparative Example 1 | 220 | 100 | 40 | 67.3 | 30 | 1.5 | 60 | 30 | 40 | 1.2 | 50/50 |
| Comparative Example 2 | 220 | 100 | 100 | 67.3 | - | | 60 | 30 | 40 | 1.2 | 50/50 |
| Comparative Example 3 | 220 | 100 | - | | 100 | 24.0 | 110 | 80 | 48 | 0.5 | 50/50 |

The disposable diapers of Examples 1 to 3 and Comparative Examples 1 and 2 were evaluated for ease of diapering and resistance to sliding down in motion in accordance with the following test methods.

1) Ease of diapering (in back-to-front way)

Eleven people who used or have used disposable diapers during child care were asked to put the diapers of the Examples, Comparative Examples and the standard product on their baby in a lying position and a standing position in a back-to-front way (the first section B of the diaper was held to the baby's back, and the fastening tapes were adhered to the landing zone on the stomach side) and to score the ease of diapering on a 100-point scale. The panelists scored the standard product, followed by the other diapers. The difference obtained by subtracting the score of the standard product from that of a tested diaper was taken as the score of that diaper indicative of ease of diapering in comparison with the standard product. The total of the scores given to each diaper by the eleven panelists are shown in Table 2.

2) Motion of sliding down on the body

A simulated infant hip model was used for testing. The model is shaped to mimic the hips and buttocks of an infant and is designed to perform a walking movement in a standing position. A diaper was put on the model in a standing position in a back-to-front way so that the top waist end of the diaper was positioned 70 mm below the top end of the model, at which the waist of the model measured 42 cm and in which the distance between the tips of the fastening tapes adhered to the landing zone was 13 cm.
The diapered model was operated to make a 5-minute walking movement at a pace of 140 steps per minute. After stopping the walking movement, the sliding distance (the distance between the original position of the top end of the diaper and the position after the walking movement) was measured. The results obtained are shown in Table 2.
The diaper of Comparative Example 2 was not tested because it did not fit the crotch of the model.

TABLE 2

| | Ease of Diapering | | Sliding Distance (mm/5min). |
|---|---|---|---|
| | on Lying Model | on Standing Model | |
| Standard Product | 0 | 0 | 20 |
| Example 1 | 30 | 95 | 13 |
| Example 2 | 70 | 115 | 15 |
| Example 3 | 35 | 45 | 16 |
| Comp. Example 1 | -105 | -35 | 19 |
| Comp. Example 2 | -120 | -270 | not measured |

The standard product and the diapers of Example 4 and Comparative Example 3 were tested for sliding distance in the same manner as described above, except that the diapers were put on backward (in a front-to-back way). The results obtained are shown in Table 3 below.

TABLE 3

| | Sliding Distance (mm/5 min) |
|---|---|
| Standard Product | 23 |
| Example 4 | 19 |
| Comp. Example 3 | 27 |

It is understood that the disposable diaper according to a first aspect of the invention is easy to put on a wearer whether the wearer is in a standing position or a lying position. It is also seen that it is reliable against leaks while worn when put on a wearer in a standing position.

**Claims**

1. A disposable diaper coinprising a liquid permeable topsheet, a liquid impermeable backsheet, a liquid retentive absorbent member interposed between the topsheet and the backsheet, a pair of longitudinal side portions and a pair of longitudinal end sections,
a fastening tape provided on each side edge of one of the longitudinal end sections thereof and a laterally extensible side part provided on each side area of a below-waist portion of the longitudinal end section having the fastening tapes, and standing gathers provided in each of the longitudinal side portions thereof, wherein
a region R having the absorbent member in a crotch section disposed therein having a low stiffnes region R1 having a flexural stiffness of 25 gf/50 mm or less in the width direction of the diaper,
the standing gathers being formed by fixing an elastic member having a fixing extension ratio of from 100% to 300 % and
the standing gathers on each longitudinal side portion having tensile characteristics, which as measured in a state of the gathers not being fixed to the diaper, wherein the tensile load required to extend the standing gathers to an effective extension ratio which is 30% lower than a fixing extension ratio, is from 20 to 120 gf, and the rate of increase of the tensile load required to extend from an extension ratio of 20% up to the effective extension ratio is 1.0-gf/% or lower.

2. The disposable diaper according to claim 1, wherein the region R comprises a high stiffness region R2 having a width of at least 50 mm across the diaper and a flexural stiffness of 25 gf/50 mm or higher in the width direction of the diaper.

3. The disposable diaper according to claim 1 or 2, wherein the low stiffness region R1 exists along each longer side edge of the diaper in the region R.

4. The disposable diaper according to any one of claims 1-3, wherein the extensible side parts each have a natural length of 20 mm or more as measured in the width direction of the diaper in a state being removed from the diaper and contracted, and the tensile characteristics of the extensible side parts are such that, when the fastening tapes are pulled apart in the width direction of the diaper to extend the extensible side parts, the tensile load at a middle extension length is from 30 to 300 gf.

5. The disposable diaper according to any one of claims 1-4, wherein the width of the crotch section as its smallest width is from 100 to 240 mm.

6. The disposable diaper according to any one of claims 1-5, wherein two portions on opposite sides of a centerline dividing the length of the diaper in equal halves have a saturated absorption capacity ratio of from 45/55 to 55/45.

**Patentansprüche**

1. Wegwerfwindel mit:

einer flüssigkeitsdurchlässigen oberen Schicht, einer flüssigkeitsundurchlässigen rückseitigen Schicht, einem zwischen der oberen Schicht und der rückseitigen Schicht angeordneten flüssigkeitszurückhaltenden absorbierenden Element, und einem Paar Längsseitenabschnitte und zwei Längsendeabschnitte, einem Verschlußband an jeder Seitenkante eines Längsendeabschnitts und jeweils einem lateral dehnbaren Seitenteil an jeder Seitenfläche eines Untertaillenabschnitts desjenigen Längsendeabschnitts, der die Verschlußbänder hat, und stehenden Falten in jedem der Längsseitenabschnitte, wobei
ein Bereich R mit dem absorbierenden Element im Schrittabschnitt einen Niedersteifigkeitsbereich R1 aufweist, der eine Biegesteifigkeit von 25gf/50mm oder weniger in Richtung der Windelbreite hat,
die stehenden Falten durch Befestigen eines elastischen Elements erzeugt sind, das ein Befestigungsdehnungsverhältnis zwischen 100% und 300% hat, und
die stehenden Falten an jedem Längsseitenabschnitt Zugeigenschaften haben, wie sie in einem nicht an der Windel befestigten Zustand der Falten gemessen werden, wobei die Zuglast, die erforderlich ist, um die stehenden Falten bis zu einem effektiven Dehnungsverhältnis, das 30% niedriger ist als ein Befestigungsdehnungsverhältnis, zu dehnen, von 20 bis 120gf ist, und die Zuglast-Steigungsrate, die erforderlich ist, um von einem Dehnungsverhältnis von 20% bis zu dem effektiven Dehnungsverhältnis zu dehnen, 1,0gf/% oder weniger ist.

**2.** Wegwerfwindel nach Anspruch 1, worin der Bereich R einen Hochsteifigkeitsbereich R2 aufweist, der eine Breite von mindestens 50mm in Querrichtung der Windel hat und eine Biegesteifigkeit von 25gf/50mm oder mehr in Richtung der Windelbreite hat.

**3.** Wegwerfwindel nach Anspruch 1 oder 2, worin in dem Bereich R der Niedersteifigkeitsbereich R1 an jeder längeren Seitenkante der Windel vorhanden ist.

**4.** Wegwerfwindel nach einem der Ansprüche 1 bis 3, worin die dehnbaren Seitenteile jeweils eine natürliche Länge von 20mm oder mehr haben, wenn gemessen in Richtung der Windelbreite in einem von der Windel abgenommenen und zusammengezogenen Zustand, und die Zugeigenschaften der dehnbaren Seitenteile derart sind, daß, wenn die Verschlußstreifen in Richtung der Windelbreite auseinandergezogen werden, um die dehnbaren Seitenteile zu dehnen, die Zuglast bei einer mittleren Dehnungslänge zwischen 30 und 300gf ist.

**5.** Wegwerfwindel nach einem der Ansprüche 1 bis 4, worin die Breite des Schrittabschnitts an seiner kleinsten Breite zwischen 100 und 240mm ist.

**6.** Wegwerfwindel nach einem der Ansprüche 1 bis 5, worin zwei Abschnitte an einander gegenüberliegenden Seiten einer Mittellinie, die die Länge der Windel in zwei gleiche Hälften teilt, ein Verhältnis ihrer gesättigten Absorptionskapazitäten zwischen 45/55 und 55/45 haben.

## Revendications

**1.** Couche jetable comprenant une feuille supérieure perméable au liquide, une feuille inférieure imperméable au liquide, un élément absorbant de rétention du liquide, intercalé entre la feuille supérieure et la feuille inférieure, une paire de portions latérales longitudinales et une paire de sections d'extrémité longitudinale,
une bande de fixation ménagée sur chaque bord latéral d'une des sections d'extrémité longitudinale et une partie latérale extensible latéralement, ménagée sur chaque zone latérale d'une portion sous la taille de la section d'extrémité longitudinale ayant les bandes de fixation, et des fronces relevées ménagées dans chacune des portions latérales longitudinales, dans lesquelles
une région R ayant l'élément absorbant dans une section en fourche, disposée à l'intérieur, ayant une zone de faible rigidité R1 ayant une rigidité à la flexion de 25 gf/50 mm ou moins dans le sens de la largeur de la couche,
les fronces relevées étant formées en fixant un élément élastique ayant un rapport d'extension de fixation de 100% à 300% et
les fronces relevées sur chaque portion latérale longitudinale ayant des caractéristiques de tension, qui sont mesurées lorsque les fronces ne sont pas fixées à la couche, dans lesquelles la charge à la tension nécessaire pour étendre les fronces relevées, selon un rapport d'extension effectif qui est de 30% inférieur à un rapport d' extension de fixation, est de 20 à 120 gf, et la vitesse d'augmentation de la charge de tension requise pour passer d'un rapport d'extension de 20% jusqu'au rapport d'extension effectif est de 1,0 gf/% ou moins.

**2.** Couche jetable selon la revendication 1, dans laquelle la zone R comprend une zone de rigidité élevée R2, d'au moins 50 mm de large, à travers la couche et une rigidité à la flexion de 25 gf/50 mm ou plus dans le sens de la largeur de la couche.

**3.** Couche jetable selon la revendication 1 ou 2, dans laquelle la région de basse rigidité R1 existe, le long de chaque bord latéral plus long de la couche dans la zone R.

**4.** Couche jetable selon l'une quelconque des revendications 1-3, dans laquelle les parties latérales extensibles ont chacune une longueur naturelle de 20 mm ou plus, comme mesuré dans la direction de la largeur de la couche, dans un état enlevé de la couche et contracté, et les caractéristiques de tension des parties latérales extensibles sont telles que, lorsque les bandes de fixation sont retirées dans le sens de la largeur de la couche, pour étendre les parties latérales extensibles, la charge de tension à une longueur d'extension moyenne est de 30 à 300 gf.

**5.** Couche jetable selon l'une quelconque des revendications 1-4, dans laquelle la largeur de la section en fourche, comme largeur plus basse, est de 100 à 240 mm.

**6.** Couche jetable selon l'une quelconque des revendications 1-5, dans laquelle deux portions sur des côtés opposés d'une ligne centrale, divisant la longueur de la couche en moitiés égales, ont un rapport de capacité d'absorption saturé de 45/55 à 55/45.

Fig.1

Fig.2

# Fig.3

# Fig.4

Fig.5(a)

Fig.5(b)

Fig.5(c)

Fig.5(d)

# Fig.6